# EUROPEAN PATENT APPLICATION

(11) **EP 4 764 502 A1**
(43) Date of publication of application: **24.06.2026**
(21) Application number: 25805595.3
(22) Date of filing: 22.05.2025
(51) Int. Cl.: G01N 33/68, G01N 33/573

(54) **USE OF BIOMARKER IN PREPARATION OF TEST KIT FOR PROGNOSTIC RISK WARNING METHOD FOR PATIENTS WITH ACUTE ST-SEGMENT ELEVATION MYOCARDIAL INFARCTION**

(30) Priority: 17.10.2024 CN 202411454596
(71) Applicant: Joinstar Biomedical Technology Co., Ltd., Hangzhou, Zhejiang 311100 (CN)
(72) Inventor: QUAN, Weiwei, Hangzhou, Zhejiang 311100 (CN); ZHANG, Junyao, Hangzhou, Zhejiang 311100 (CN); WANG, Yueying, Hangzhou, Zhejiang 311100 (CN); HONG, Longbin, Hangzhou, Zhejiang 311100 (CN); ZHU, Tianqi, Hangzhou, Zhejiang 311100 (CN); DONG, Ming, Hangzhou, Zhejiang 311100 (CN)
(74) Representative: Acapo Onsagers AS
(86) International application number: PCT/CN2025/096563
(87) International publication number: WO 2026/081480

(57) **Abstract**

Disclosed is a use of a biomarker in the preparation of a kit used in a method for early warning of a prognosis risk in a patient with acute ST-segment elevation myocardial infarction. The biomarker comprises HBP. The present application can effectively evaluate whether patients with acute ST-segment elevation myocardial infarction will experience poor prognosis within 30 days after PCI by monitoring changes in HBP levels, thereby achieving early identification and timely clinical intervention for high-risk patients, improving the response speed and effect of treatment, reducing the incidence of adverse cardiovascular events, and ultimately improving prognosis of patients.

## Description

### TECHNICAL FIELD

The present application belongs to the technical field of medical biology, and in particular relates to a use of a biomarker in the preparation of a kit used in a method for early warning of a prognosis risk in a patient with acute ST-segment elevation myocardial infarction and a kit thereof, and specifically relates to a use of HBP in early warning of a prognosis risk in a patient with acute ST-segment elevation myocardial infarction.

### BACKGROUND

Acute myocardial infarction (AMI) is an acute and critical disease based on coronary atherosclerotic lesions. Atherosclerotic plaques rupture and thrombosis lead to acute occlusion of the affected culprit blood vessels, resulting in myocardial ischemia and necrosis. It is one of the main causes of death and disability among cardiovascular diseases. In the past decade, the country has actively carried out the construction of chest pain centers, which has improved people's understanding of myocardial infarction and the ability to treat AMI, greatly improving the prognosis. However, AMI patients still have a high incidence of poor prognosis, including major cardiovascular events (MACCE) such as reinfarction, acute heart failure, cardiogenic shock, and stroke. It has been found that poor prognosis of AMI is closely related to ventricular remodeling. A series of ventricular remodeling processes can occur immediately after acute myocardial infarction, causing changes in cardiac structure and reduced cardiac function. Inflammatory response plays a crucial role in the process of ventricular remodeling. The more obvious the inflammatory response and ventricular remodeling after acute myocardial infarction, the more likely it is to cause malignant cardiovascular events such as acute heart failure, cardiogenic shock, heart rupture, and stroke.

The first to reach the infarction region in the inflammatory response after acute myocardial infarction is neutrophils. Tamura *et al.* confirmed that neutrophil count in WBC is correlated with left ventricular end-systolic diameter and left ventricular end-diastolic diameter.

In recent years, a new type of inflammatory factor, heparin binding protein (HBP), has been discovered. It is synthesized by neutrophils and stored in corresponding locations. Once neutrophils are activated, they release a large amount of HBP, which participates in various inflammatory processes as an inflammatory mediator.

When HBP is rapidly secreted into the blood, it has the effect of chemotaxis on monocytes, and monocytes activated and chemotaxed by HBP cause inflammatory responses that play an important role in the formation and progress of atherosclerotic cardiovascular disease (ASCVD).

It has been proved that HBP can induce and promote the massive release of inflammatory mediators such as tumor necrosis factor-a (TNF-α) and interleukin-1 (IL-1), and the correlation between TNF-α, IL-1 and ASCVD has been fully confirmed by previous studies. This indicates a close relationship between HBP and ASCVD. For patients with acute ST-segment elevation myocardial infarction (acute ST elevated myocardial infarction, STEMI), even if they receive primary PCI treatment, they still face a high risk of poor prognosis after pPCI. Patients with acute myocardial infarction continue to have a risk of recurrence and death within 1 year after discharge, especially within the first month after discharge, when the risk is highest, accounting for 30% of the total MACCE. Therefore, early identification of high-risk patients after myocardial infarction, timely treatment and early intervention can help reduce the adverse prognosis of these high-risk myocardial infarction patients.

Although previous studies have shown that the concentration of HBP in STEMI patients is closely related to poor prognosis during hospitalization, with high levels of HBP indicating poor prognosis, this association has not been widely validated, and whether HBP levels during hospitalization are associated with adverse events in myocardial infarction patients within one month after discharge has never been confirmed. In view of this, it is crucial to find a biomarker that can accurately identify individuals with poor prognosis in the early stages of acute myocardial infarction, because this will help to achieve early identification and intensive treatment of patients at high risk of myocardial infarction, thereby improving the long-term survival rate of patients with myocardial infarction and improving the short- and long-term prognosis of patients with myocardial infarction.

### SUMMARY

In view of this, a use of a biomarker in the preparation of a kit used in a method for early warning of a prognosis risk in a patient with acute ST-segment elevation myocardial infarction is proposed in the present application, aiming to effectively predict the possible deterioration of the STEMI patient's condition using HBP levels, ensure timely clinical intervention measures, and ultimately realize the purpose of improving the prognosis of such high-risk patient.

In a first aspect, the present application provides a use of a biomarker in the preparation of a kit used in a method for early warning of a prognosis risk in a patient with acute ST-segment elevation myocardial infarction, wherein the biomarker comprises HBP.

Heparin-binding protein (HBP), also known as azurocidin or cationic antimicrobial protein of 37 KDa (CAP37), is a secreted granulin located in a polymorphonuclear neutrophil (PMN) secretory vesicle and an azurophilic granule. A sequence of the HBP is publicly obtainable. For example, the sequence of the HBP is obtained by an accession number NP 001691REGION: 27. . . 248 of a national center of biotechnology information (NCBI).

By adopting the above technical solution, the present application provides a use of a biomarker in the preparation of a kit used in a method for early warning of a prognosis risk in a patient with acute ST-segment elevation myocardial infarction. By monitoring changes in HBP levels, the possibility of deterioration of the patient's condition can be effectively evaluated, thereby achieving early identification of the high-risk patient and timely clinical intervention, achieving the technical effect of improving the response speed and effect of treatment, reducing the incidence of adverse cardiovascular events and improving prognosis of the patient.

As an inflammatory marker previously used mainly to evaluate bacterial infection and sepsis, HBP was used for the first time in this study to evaluate myocardial injury in STEMI patients, and it is currently the largest sample size related to the prognosis of myocardial infarction. This study demonstrated for the first time that HBP can be used independently for the prognosis evaluation of STEMI patients, both in terms of adverse events during hospitalization and in the near term after discharge, and for the first time revealed the close association between elevated HBP levels and the occurrence of heart failure after myocardial infarction, indicating that HBP can be used as an effective tool to predict the occurrence of heart failure after myocardial infarction. The researchers of the present application found that on days 1, 2, and 3 after percutaneous coronary intervention (PCI) in STEMI patients, HBP levels were an independent risk factor for the occurrence of heart failure events within one month after PCI. Further research has shown that the HBP level on day 3 after PCI in STEMI patients has good discriminatory value for the occurrence of heart failure events within one month after discharge.

In summary, the present application provides a new method for assessing the prognosis risk in a patient with acute ST-segment elevation myocardial infarction (STEMI) by monitoring changes in HBP levels. This technical solution enables clinicians to effectively assess the possibility of deterioration of the patients' condition, especially for those patients who are still at high risk after percutaneous coronary intervention (PCI), and can achieve early identification and timely clinical intervention, thereby improving the response speed and effect of treatment, reducing the incidence of adverse cardiovascular events, and ultimately improving the patient's prognosis. In addition, this study not only used HBP as an independent marker for the prognosis assessment of STEMI patients for the first time, but also revealed the close association between elevated HBP levels and the occurrence of heart failure after myocardial infarction, indicating that HBP has potential value in predicting the occurrence of heart failure after myocardial infarction. In particular, the HBP level on day 3 after PCI was found to be able to better distinguish the risk of heart failure events within one month, which provides valuable reference information for clinical practice.

The method for early warning of a prognosis risk comprises detecting the concentration of the biomarker in the plasma of the patient with acute ST-segment elevation myocardial infarction 24 hours, 48 hours and 72 hours after percutaneous coronary intervention, to determine whether the patient with acute ST-segment elevation myocardial infarction has a risk of poor prognosis after percutaneous coronary intervention; the poor prognosis comprises the occurrence of one of the following cardiovascular events: reinfarction, acute heart failure, cardiogenic shock and stroke.

By adopting the above technical solution, the present application can effectively evaluate the prognosis risks of patients after PCI, timely identify high-risk patients who may have a poor prognosis, and provide a scientific basis for clinicians to take corresponding preventive and intervention measures, improve treatment effects, reduce the incidence of adverse cardiovascular events, and ultimately improve the prognosis of patients.

When the concentration of HBP in the plasma of patients with acute ST-segment elevation myocardial infarction within 72 hours is greater than 19.6ng/mL, it is determined that patients with acute ST-segment elevation myocardial infarction who receive primary PCI treatment have a risk of poor prognosis within 30 days after discharge.

The biomarker further comprises hs-CRP.

By adopting the above technical solution, the present application can further enhance the accuracy of assessing the prognosis risks of patients with acute ST-segment elevation myocardial infarction (STEMI). When HBP is used in combination with hs-CRP, especially on day 3 after primary PCI (pPCI) treatment in STEMI patients, the predictive ability of HBP levels for heart failure events is improved in statistical analysis, providing more accurate prognostic information for clinicians and helping to achieve early identification and timely intervention of high-risk patients to improve treatment outcomes and prognosis.

Optionally, the kit is configured to detect a change in HBP concentration in the patient with acute ST-segment elevation myocardial infarction.

Optionally, the change in HBP concentration is used as an early warning indicator for the prognosis risk of the patient with acute ST-segment elevation myocardial infarction.

Optionally, the prognosis risk comprises one or a combined risk of reinfarction, acute heart failure, cardiogenic shock and stroke.

Optionally, the kit is configured to detect the change in HBP concentration in the patient with acute ST-segment elevation myocardial infarction by detecting an in vitro sample from the patient.

Optionally, the in vitro sample is plasma.

Optionally, the biomarker is used in early warning of a prognosis risk in a patient with acute ST-segment elevation myocardial infarction by the following method:
S1, sample collection: collecting a plasma sample from the patient with acute ST-segment elevation myocardial infarction using a test tube containing sodium citrate anticoagulant; wherein the ratio of the sodium citrate anticoagulant to the plasma sample is 1:9;
S2, sample processing: during the process of separating plasma, taking care to avoid inhaling any white blood cells, as white blood cells can release high levels of HBP, which may interfere with the detection results;
S3, detection instrument and method: detecting HBP levels using Jet-iStar 3000 fully automatic immunoassay analyzer (produced by Zhonghan Shengtai Biotechnology Co., Ltd., located in Zhejiang, China); wherein dry fluorescence immunoassay is used for detection in the instrument; and
S4, operation procedure: taking 50 microliters (µl) of the processed plasma sample described in step S2, adding it to the Jet-iStar 3000 fully automatic immunoassay analyzer described in step S3, and incubating for 18 minutes, after which the instrument will automatically detect and report the level of HBP.

By adopting the above technical solution, the present application can effectively evaluate the prognosis risks of patients with acute ST-segment elevation myocardial infarction who receive primary PCI treatment within 30 days after discharge, thereby achieving early identification and timely clinical intervention of high-risk patients, improving the response speed and effect of treatment, reducing the incidence of adverse cardiovascular events, and ultimately improving prognosis of patients.

In a second aspect, the present application provides a kit used in a method for early warning of a prognosis risk in a patient with acute ST-segment elevation myocardial infarction, wherein the kit comprises a reagent for extracting HBP and/or a reagent for detecting HBP.

By adopting the above technical solutions, the kit can support clinical laboratories to quickly and accurately measure the concentration of HBP in patients, thereby helping to assess the risk of poor prognosis of the patients within 30 days after PCI. This standardized kit not only simplifies the testing process and improves the testing efficiency, but also ensures the consistency and reliability of the test results, providing clinicians with a powerful tool to achieve early identification and timely intervention of high-risk patients, thereby improving the treatment effect and prognosis of patients.

In summary, the present application has at least one of the beneficial technical effects as follows:
1. The present application provides a use of a biomarker in the preparation of a kit used in a method for early warning of a prognosis risk in a patient with acute ST-segment elevation myocardial infarction by monitoring changes in HBP levels. This technical solution enables clinicians to effectively assess the possibility of deterioration of the patients' condition, especially for those patients who are still at high risk after percutaneous coronary intervention (PCI), and can achieve early identification and timely clinical intervention, thereby improving the response speed and effect of treatment, reducing the incidence of adverse cardiovascular events, and ultimately improving the patients' prognosis. In addition, this study not only used HBP as an independent marker for the prognosis assessment of STEMI patients for the first time, but also revealed the close association between elevated HBP levels and the occurrence of heart failure after myocardial infarction, indicating that HBP has potential value in predicting the occurrence of heart failure after myocardial infarction. In particular, the HBP level on day 3 after PCI was found to be able to better distinguish the risk of heart failure events within one month, which provides valuable reference information for clinical practice.
2. The present application can effectively evaluate the prognosis risks of patients with acute ST-segment elevation myocardial infarction who receive primary PCI treatment within 30 days after discharge, thereby achieving early identification and timely clinical intervention of high-risk patients, improving the response speed and effect of treatment, reducing the incidence of adverse cardiovascular events, and ultimately improving prognosis of patients.

### DESCRIPTION OF THE DRAWINGS

FIG. 1 shows a graph of HBP fluctuations before and after pPCI in STEMI patients in the present application;
FIG. 2 shows a graph of ROC curve analysis for STEMI patients;
FIG. 3 shows a graph of the dose-response relationship between HBP and in-hospital adverse outcomes;
FIG. 4 shows a graph of the dose-response relationship between HBP and adverse outcomes 30 days after discharge;
FIG. 5 shows a Kaplan-Meier survival curve;
FIG. 6 shows a graph of the correlation between the peak HBP levels before PCI and on days 1, 2, and 3 after PCI and the echocardiographic LVEF of STEMI patients 1 month after PCI;
FIG. 7 shows a graph of ROC curve analysis.

### DETAILED DESCRIPTION

In order to make the purpose, technical solution and advantages of the present application more clear, the present application is further described in detail below in conjunction with the examples. It should be understood that the specific examples described here are only used to explain the present application and are not used to limit the present application.

The following specific examples will be used to explain the solution of the present application. Unless otherwise specified, the raw materials used in the following examples are all from ordinary commercially available products, and the devices or equipment used are purchased from conventional market sales channels.

The inclusion criteria are as follows:
Patients eligible for inclusion in this study must meet all of the following criteria:
(1) written informed consent must be obtained before any assessment is conducted;
(2) male or female patients aged 18 or above; and
(3) diagnosed as spontaneous AMI according to the generally accepted definition of myocardial infarction*.

Spontaneous AMI is defined as a clinical setting in which there is evidence of myocardial necrosis consistent with myocardial ischemia resulting from a major coronary event. In these circumstances, spontaneous AMI must be diagnosed according to the following criteria:
elevated and/or decreased myocardial enzymes (cardiac troponin, cTn, or CK-MB) were detected, with at least one detection value exceeding the 99th percentile of the upper limit of normal (URL) or the local laboratory's MI diagnostic critical value, and at least one of the following evidence of myocardial ischemia is present:
(1) ischemic discomfort or other ischemic symptoms;
(2) ECG features of STEMI, including significant ST-T changes that are new or speculated to be new; and
(3) the presence of new pathological Q waves or left bundle branch block on ECG (*Patients whose spontaneous MI was secondary to another medical illness, such as anemia, hypotension, or arrhythmia, or whose spontaneous MI was thought to be caused by coronary vasospasm and whose coronary arteries were normal, were not eligible).

Patients who had clinical manifestations related to Takotsubo cardiomyopathy also did not meet the inclusion criteria (this MI visit refers to the time when the patient visited the emergency room/emergency department (ER/ED), was admitted to the intensive care unit/cardiovascular care unit (CCU) or hospital ward, etc., for treatment of this MI).

The exclusion criteria are as follows:
Patients who meet any of the following criteria do not meet the eligibility requirements for inclusion in this study:
(1) have a known history of chronic heart failure;
(2) cardiogenic shock occurred within 24 hours before enrollment;
(3) persistent clinical heart failure before enrollment;
(4) stroke or transient ischemic attack occurred within one month before enrollment; and
(5) before enrollment, the patient had severe infection, trauma, hematological disease, surgical operation, and other diseases assessed by researchers as potentially interfering with HBP measurement.

The research subjects are as follows:
This study lasted for 125 days and was approved by the Ethics Committee of Ruijin Hospital Affiliated to Shanghai Jiao Tong University School of Medicine (2018-183), and informed consent was obtained from the participants.

251 STEMI patients who visited Ruijin Hospital Affiliated with Shanghai Jiao Tong University School of Medicine from August 2023 to April 2024 were included, excluding 5 patients with a history of heart failure, 15 patients with cardiogenic shock within 24 hours of admission, 1 patient with stroke within 1 month before admission, and 15 patients with severe infections. Finally, 215 STEMI patients were included in this study.

### Experimental testing:

### Part I: Quantitative detection of HBP:

Plasma specimens anticoagulated with sodium citrate in a ratio of 1:9 were used for testing.

When separating plasma, be careful not to inhale any white blood cells to prevent them from releasing high levels of HBP. A 50 µl plasma sample was tested using a Jet-iStar3000 fully automatic immunoassay analyzer (Zhonghan Shengtai Biotechnology Co., Ltd. in Zhejiang, China), and the HBP level was measured after incubation for 18 min (dry fluorescence immunoassay).

The statistical analysis related to the present application comprises the following:
(1) Continuous variables that conform to normal distribution were expressed as mean ± standard deviation, and continuous variables that do not conform to normal distribution were expressed as median (25% quantile, 75% quantile);
(2) Categorical variables were expressed as frequency (percentage);
(3) Correlation analysis was performed using Pearson or Spearman correlation analysis and a scatter plot was drawn;
(4) Construct receiver operating characteristic (ROC) curves and area under the curve (AUC) to illustrate various cutoff levels of HBP;
(4) Cox regression analysis was used to analyze relevant risk factors and calculate the hazard ratio (HR) and 95% confidence interval (CI);
(5) Kaplan-Meier survival analysis was performed;
(6) P < 0.05 was considered statistically significant;
(7) Data were analyzed and graphs were drawn using R (Bell Laboratories version 4.0.0), GraphPad Prism 8.0.2 (GraphPad Software, San Diego, CA, USA), and IBM SPSS (Statistics for Windows Version 22.0, IBM, Chicago, IL, USA).

Part II: The overall information of the participants is as follows:
(1) The overall information of the participants in this study is shown in Table 1.

**Table 1. Participant overall information**

| N | | Patients (N=215) |
|---|---|---|
| Demographic characteristics | Age, years | 64.17±12.26 |
| | Gender, male (%) | 170 (79.10) |
| | BMI, kg/m² | 24.95±3.65 |
| | Heart rate, bpm | 83.71±15.61 |
| | Systolic blood pressure, mmHg | 131.63±78.05 |
| | Diastolic blood pressure, mmHg | 77.58±13.49 |
| | Current smoking, n (%) | 35 (16.28) |
| History of disease | Diabetes mellitus, n (%) | 60 (27.91) |
| | Hypertension, n (%) | 147 (68.37) |
| | Chronic kidney disease, n (%) | 15 (6.98) |
| Killip grade | I, n (%) | 186 (86.51) |
| | II, n (%) | 13 (6.05) |
| | III-IV, n (%) | 16 (7.44) |
| Culprit vessel | Anterior descending branch, n (%) | 103 (47.91) |
| | Circumflex branch, n (%) | 47 (21.86) |
| | Right coronary artery, n (%) | 65 (30.23) |

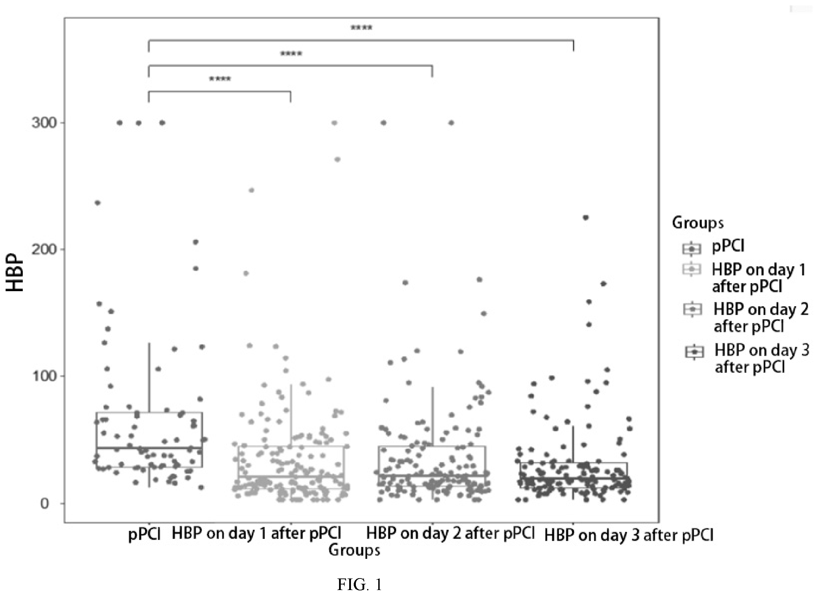

HBP fluctuations before and after pPCI in STEMI patients:
The HBP fluctuations before and after pPCI in STEMI patients are shown in FIG. 1.

Analysis of the results in FIG. 1: HBP level in STEMI patients before pPCI was significantly increased, which was 43.43 (28.60, 71.96) ng/mL;
The HBP levels on days 1, 2, and 3 after pPCI were 21.24 (11.10, 45.21) ng/mL, 21.86 (13.49, 45.84) ng/mL, and 19.54 (12.34, 32.28) ng/mL, respectively.

In conclusion, the HBP levels of STEMI patients before and 72 hours after pPCI were higher than the normal range.

Compared with the HBP level before pPCI, the HBP level after pPCI decreased significantly, and the results were statistically significant (p<0.001).

(2) The predictive value of HBP for in-hospital adverse events in STEMI patients

The results of univariate and multivariate logistic regression are showed in Table 2. From the test data provided in Table 2, it can be seen that the HBP levels of STEMI patients on days 1, 2, and 3 after pPCI, as well as the peak HBP levels, are independent risk factors for the risk of in-hospital adverse events in STEMI patients.

**Table 2. Logistic univariate and multivariate analysis of the predictive value of HBP for in-hospital events**

| HBP before pPCI | Uncorrected OR (%95 CI) | P | Model 1 OR (%95 CI) | P | Model 2 OR (%95 CI) | P |
|---|---|---|---|---|---|---|
| HBP on day 1 after pPCI | 1.004 (0.997 to 1.012) | 0.254 | 1.008 (0.999 to 1.017) | 0.089 | 1.016 (1.000 to 1.031) | 0.045 |
| HBP on day 2 after pPCI | 1.008 (1.000 to 1.016) | 0.047 | 1.007 (0.998 to 1.015) | 0.111 | 1.015 (1.004 to 1.026) | 0.009 |
| HBP on day 3 after pPCI | 1.012 (1.003 to 1.022) | 0.008 | 1.012 (1.003 to 1.022) | 0.009 | 1.015 (1.003 to 1.027) | 0.014 |
| HBP peak value | 1.015 (1.003 to 1.027) | 0.011 | 1.015 (1.003 to 1.027) | 0.013 | 1.015 (1.001 to 1.029) | 0.032 |
| Δ1 HBP | 1.007 (1.002 to 1.013) | 0.013 | 1.007 (1.002 to 1.013) | 0.013 | 1.012 (1.005 to 1.020) | 0.001 |
| Δ2 HBP | 1.005 (0.997 to 1.014) | 0.238 | 1.013 (1.001 to 1.025) | 0.030 | 1.086 (0.987 to 1.196) | 0.091 |
| Δ3 HBP | 1.006 (0.996 to 1.015) | 0.266 | 1.011 (0.999 to 1.024) | 0.078 | 1.042 (0.999 to 1.086) | 0.054 |

| | | | | | | |
|---|---|---|---|---|---|---|
| Δ 1HBP was calculated as the level assessed before PCI minus the HBP level on day 1 after PCI; Δ 2HBP was calculated as the level assessed before PCI minus the HBP level on day 2 after PCI; Δ 3HBP was calculated as the level assessed before PCI minus the HBP level on day 3 after PCI; Model 1 was used for correcting age and gender; Model 2 was used for correcting BMI, smoking, history of hypertension, history of diabetes, history of dyslipidemia, KILLIP grade, WBC, hsCRP and LVEF at baseline for age and gender. | | | | | | |

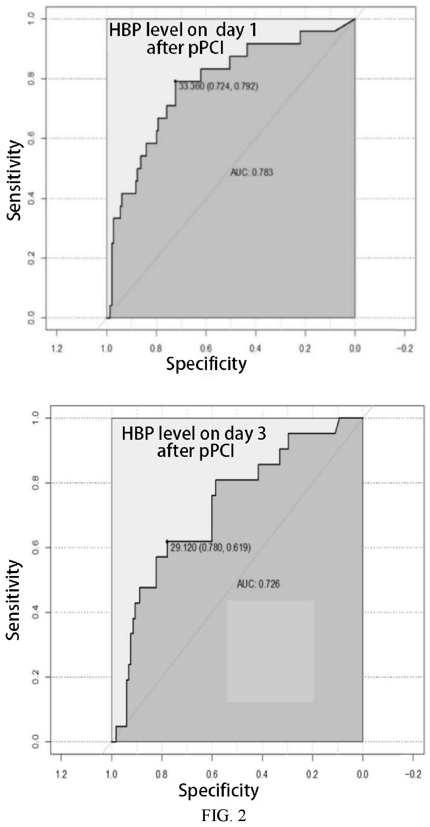

The ROC curve analysis, as shown in FIG. 2, indicates that the HBP level on day 1 after PCI in STEMI patients has good discriminatory value for in-hospital adverse events.

A cut-off level greater than 33.36 ng/mL has a sensitivity of 72.4% and a specificity of 79.2% in predicting in-hospital adverse events, with an area under the curve of 0.78.

In addition, the HBP level on day 3 after pPCI in STEMI patients also has good discriminatory value for in-hospital adverse events.

A cut-off level greater than 29.12 ng/mL has a sensitivity of 78.0% and a specificity of 61.9% in predicting in-hospital adverse events

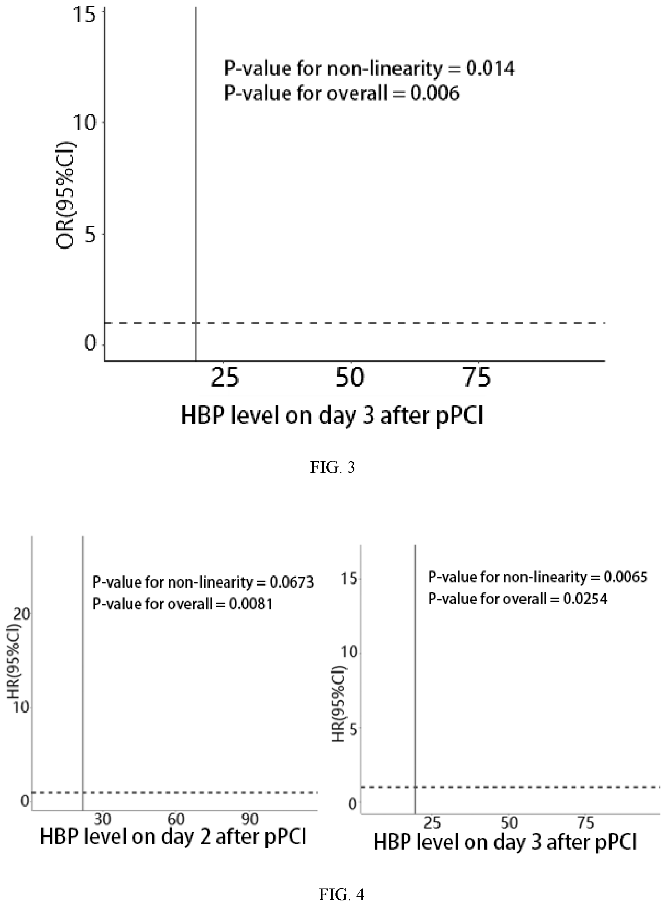

(3) Further explore the dose-response relationship between HBP and adverse in-hospital outcomes, as shown in FIG. 3.

Analysis of the results in FIG. 3: the inventors found that when the HBP level of STEMI patients was less than 19.6 ng/mL on day 3 after pPCI, the risk of in-hospital adverse events was not significant. When the HBP level was greater than 19.6 ng/mL, the risk of in-hospital adverse events in patients increased significantly with the increase of the HBP level.

(4) The predictive value of HBP for adverse events in STEMI patients 30 days after discharge

The results of univariate and multivariate COX regression showed that HBP levels on days 1, 2, and 3 after pPCI, as well as the peak HBP levels, were independent risk factors for the risk of adverse events in STEMI patients during the 30-day follow-up (independent of hsCRP, LVEF, etc.).

The COX univariate and multivariate analysis of the predictive value of HBP for 30-day adverse events in STEMI patients is shown in Table 3.

**Table 3. COX univariate and multivariate analysis table of the predictive value of HBP for 30-day adverse events in STEMI patients**

| | Uncorrected HR(CI) | P | Model 1 HR (%95 CI) | P | Model 2 HR (%95 CI) | P |
|---|---|---|---|---|---|---|
| HBP before pPCI | 1.003 (0.997 to 1.010) | 0.304 | 1.005 (0.998 to 1.011) | 0.178 | 1.023 (0.997 to 1.044) | 0.350 |
| HBP on day 1 after pPCI | 1.007 (1.001 to 1.013) | 0.024 | 1.005 (0.999 to 1.011) | 0.130 | 1.009 (1.001 to 1.018) | 0.035 |
| HBP on day 2 after pPCI | 1.006 (1.002 to 1.011) | 0.007 | 1.007 (1.002 to 1.012) | 0.006 | 1.008 (1.001 to 1.015) | 0.018 |
| HBP on day 3 after pPCI | 1.009 (1.002 to 1.015) | 0.011 | 1.009 (1.002 to 1.015) | 0.012 | 1.013 (1.003 to 1.024) | 0.012 |
| HBP peak value | 1.005 (1.001 to 1.009) | 0.016 | 1.005 (1.001 to 1.009) | 0.016 | 1.008 (1.003 to 1.013) | 0.002 |
| Δ1 HBP | 1.005 (0.998 to 1.011) | 0.194 | 1.007 (1.000 to 1.014) | 0.050 | 1.121 (0.969 to 1.297) | 0.123 |
| Δ2 HBP | 1.005 (0.997 to 1.013) | 0.216 | 1.007 (0.998 to 1.016) | 0.152 | 1.149 (0.841 to 1.569) | 0.384 |
| Δ3 HBP | 1.004 (0.995 to 1.014) | 0.375 | 1.007 (0.996 to 1.019) | 0.222 | 1.209 (0.996 to 1.447) | 0.380 |

| | | | | | | |
|---|---|---|---|---|---|---|
| Δ 1HBP was calculated as the level assessed before pPCI minus the HBP level on day 1 after pPCI; Δ 2HBP was calculated as the level assessed before pPCI minus the HBP level on day 2 after pPCI; Δ 3HBP was calculated as the level assessed before pPCI minus the HBP level on day 3 after pPCI; Model 1 was corrected for age and gender; Model 2 was corrected for BMI, smoking, history of hypertension, history of diabetes, history of dyslipidemia, KILLIP grade, WBC, hsCRP and LVEF at baseline for age and gender. | | | | | | |

(5) Further explore the dose-response relationship between HBP and adverse outcomes 30 days after discharge, as shown in FIG. 4

Analysis of the results in FIG. 4: the HBP level of STEMI patients on day 2 after pPCI was less than 21.80 ng/mL. The risk of adverse events within 30 days was not significant. When the HBP level was greater than 21.80 ng/mL, the risk of adverse events within 30 days increased with the increase of the HBP level.

When the HBP level of STEMI patients was less than 19.63 ng/mL on day 3 after pPCI, the risk of adverse events within 30 days was not significant. When the HBP level was greater than 19.63 ng/mL, the risk of adverse events within 30 days increased with the increase of the HBP level.

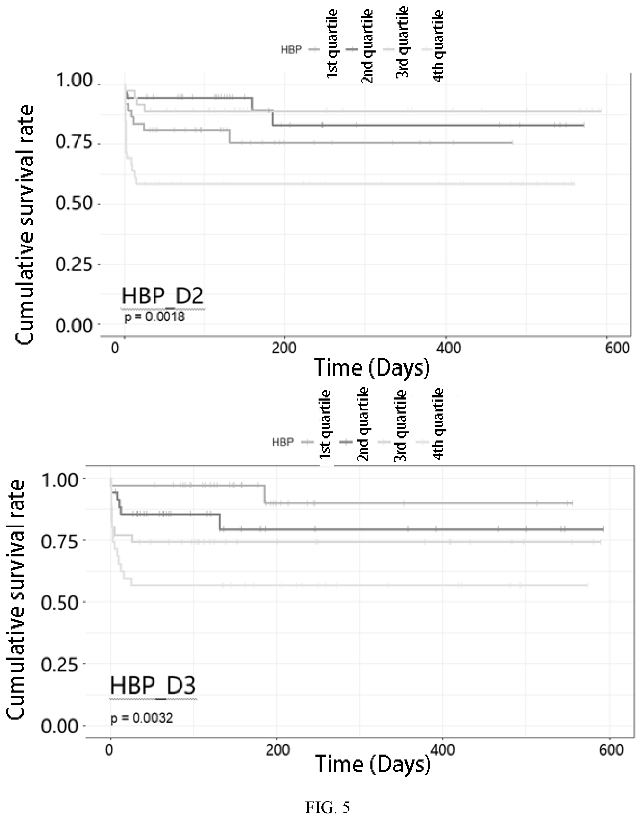

(6) The Kaplan-Meier survival curve is shown in FIG. 5.

Analysis of the results in FIG. 5: the incidence of adverse events within 30 days was 12 times higher in STEMI patients with the highest quartile of HBP levels on days 2 and 3 after pPCI compared to those with the lowest quartile. There was no statistically significant difference in HBP levels before and on day 1 after pPCI. HBP is expected to become an effective evaluation tool for the long-term prognosis of myocardial infarction patients after discharge.

(7) Correlation between HBP and left ventricular ejection fraction (LVEF)

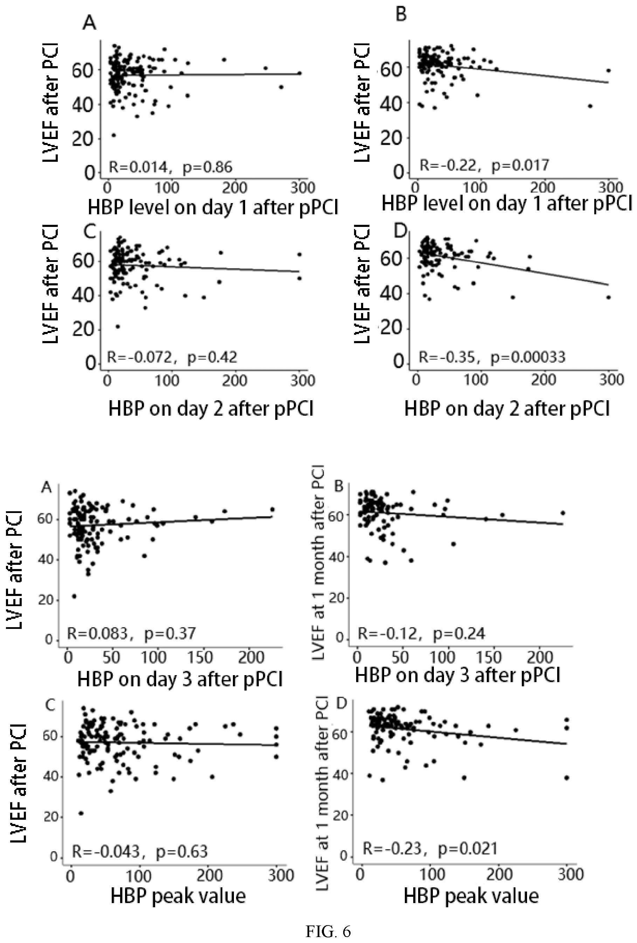

The correlation between the HBP levels before pPCI and on days 1, 2, and 3 after pPCI and the peak HBP levels in STEMI patients and the echocardiographic LVEF at 1 month after pPCI is shown in FIG. 6.

Analysis of the results in FIG. 6: HBP level and HBP peak value of STEMI patients on day 1 after pPCI were negatively correlated with ejection fraction at 1 month during follow-up after pPCI (r=-0.22, p=0.017);

The HBP level on day 2 after pPCI (r=-0.35, p=0.0003) and the HBP peak value (r=-0.23, p=0.021) were negatively correlated with the LVEF at 1 month during follow-up after pPCI.

In addition, the results of univariate linear regression showed (see Table 4) that the HBP levels of STEMI patients on days 1 and 2 after pPCI and HBP peak values were negatively correlated with LVEF at 1 month during follow-up after pPCI.

**Table 4. Univariate linear regression analysis of the relationship between HBP level and peak value during hospitalization and left ventricular ejection fraction 1 day and 1 month after pPCI in STEMI patients**

| | LVEF within 24 hours after pPCI | | | LVEF within 1 month after pPCI | | |
|---|---|---|---|---|---|---|
| | *β* | *95% CI for β* | *p* | *β* | *95% CIfor β* | *p* |
| HBP before pPCI | -0.052 | (-0.039 to 0.026) | 0.694 | 0.069 | (-0.022 to 0.036) | 0.630 |
| HBP on day 1 after pPCI | 0.028 | (-0.034 to 0.047) | 0.767 | -0.257 | (-0.096 to -0.010) | 0.015 |
| HBP on day 2 after pPCI | -0.095 | (-0.052 to 0.016) | 0.304 | -0.364 | (-0.099 to -0.031) | <0.001 |
| HBP on day 3 after pPCI | 0.084 | (-0.025 to 0.068) | 0.371 | -0.121 | (-0.072 to 0.019) | 0.247 |
| HBP peak value | -0.042 | (-0.029 to 0.018) | 0.624 | -0.233 | (-0.054 to -0.006) | 0.013 |
| Δ1 HBP | -0.136 | (-0.050 to 0.020) | 0.389 | 0.150 | (-0.018 to 0.046) | 0.376 |
| Δ2 HBP | -0.041 | (-0.044 to 0.033) | 0.787 | 0.060 | (-0.026 to 0.039) | 0.704 |
| Δ3 HBP | 0.033 | (-0.034 to 0.043) | 0.824 | 0.124 | (-0.022 to 0.051) | 0.428 |

| | | | | | | |
|---|---|---|---|---|---|---|
| Δ 1HBP was calculated as the level assessed before pPCI minus the HBP level on day 1 after pPCI; Δ 2HBP was calculated as the level assessed before pPCI minus the HBP level on day 2 after pPCI; Δ 3HBP was calculated as the level assessed before pPCI minus the HBP level on day 3 after pPCI. | | | | | | |

The predictive value of HBP in the occurrence of heart failure events in STEMI patients after myocardial infarction

The results of COX regression (see Table 5) showed that HBP levels on days 1, 2, and 3 after pPCI in STEMI patients were independent risk factors for the risk of heart failure events during follow-up after PCI.

**Table 5. COX univariate analysis of the predictive value of HBP for the occurrence of heart failure events in STEMI patients within 30 days after discharge**

| N/A | Univariate COX regression | | |
|---|---|---|---|
| | HR | 95% CI | p |
| HBP before pPCI | 0.988 | 0.963 to 1.014 | 0.355 |
| HBP on day 1 after pPCI | 1.010 | 1.004 to 1.016 | 0.001 |
| HBP on day 2 after pPCI | 1.008 | 1.003 to 1.014 | 0.002 |
| HBP on day 3 after pPCI | 1.011 | 1.003 to 1.019 | 0.006 |
| HBP peak value | 1.004 | 0.999 to 1.009 | 0.126 |

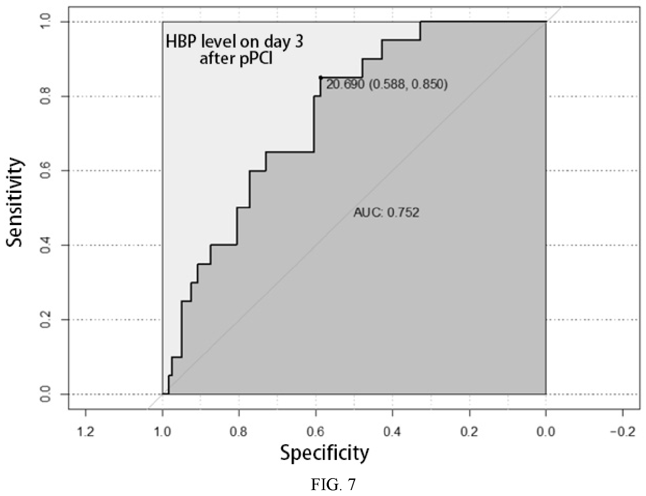

(8) In addition, the ROC curve analysis is shown in FIG. 7.

Analysis of the results in FIG. 7: HBP level on day 3 after pPCI in STEMI patients have good discriminatory value for heart failure events. The cut-off level of 20.69 ng/mL had a sensitivity of 58.8% and a specificity of 85.0% in predicting in-hospital adverse events, with an AUC of 0.75.

(9) The predictive value of HBP combined with hs-CRP for heart failure events

As shown in Table 6, when used in combination with hs-CRP, the HBP level on day 3 after pPCI in STEMI patients provided improved prediction of heart failure events in c-statistical analysis.

**Table 6. Accuracy of cTnI, hs-CRP and HBP in predicting the risk of heart failure in STEMI patients**

| N/A | C-statistic | P-value | IDI (95%CI) | P-value |
|---|---|---|---|---|
| Log2 cTnI | 0.623 | NA | Ref | NA |
| Log2 HBP | 0.680 | 0.263 | 0.030 (0.001, 0.058) | 0.044 |
| Log2 hs-CRP | 0.612 | 0.807 | 0.007 (-0.015, 0.029) | 0.536 |
| Log2 hs-CRP + Log2 HBP | 0.675 | 0.126 | 0.042 (0.020, 0.064) | <0.001 |
| Log2 cTnI+ Log2 hs-CRP + Log2 HBP | 0.675 | 0.158 | 0.059 (0.031, 0.086) | <0.001 |

In summary, the applicant found that the HBP levels of STEMI patients before and within 72 hours after pPCI were higher than normal. Before and 24 hours to 72 hours after pPCI, HBP gradually decreased, significantly lower than before pPCI. The HBP level of plasma can be used as an independent risk factor to evaluate poor prognosis events in the hospital and 30 days after discharge.

In addition, HBP was negatively correlated with the cardiac function level of STEMI patients one month after pPCI. The higher the HBP level, the worse the cardiac function. It is an independent risk factor for heart failure events after myocardial infarction within 30 days.

The HBP level combined with hs-CRP on day 3 after pPCI in STEMI patients improved the predictive value of heart failure events within one month after pPCI.

As an inflammatory marker, HBP has previously focused on the research of bacterial infections and sepsis. At present, few scholars in China and abroad have applied HBP to evaluate myocardial injury in patients with acute myocardial infarction. This study is the first to explore the dynamic changes in HBP levels before and 72 hours after pPCI in STEMI patients, as well as their relationship with the prognosis of myocardial infarction. It is currently STEMI related HBP research work with the largest sample size. This study demonstrates for the first time that HBP can be independently used for prognostic evaluation of STEMI patients, whether it is in-hospital adverse events or recent poor prognosis. It also reveals for the first time that elevated HBP levels are closely related to the occurrence of heart failure after myocardial infarction, and can serve as an effective predictive tool for heart failure after myocardial infarction.

The above description is only the preferred embodiment of the present application and is not intended to limit the present application. Any modifications, equivalent substitutions, and improvements made within the principles of the present application should be included in the scope of protection of the present application.

## Claims

1. Use of a biomarker in the preparation of a kit used in a method for early warning of a prognosis risk in a patient with acute ST-segment elevation myocardial infarction, wherein the biomarker comprises HBP.

2. The use according to claim 1, wherein the biomarker further comprises hs-CRP.

3. The use according to claim 1, wherein the kit is configured to detect a change in HBP concentration in the patient with acute ST-segment elevation myocardial infarction.

4. The use according to claim 3, wherein the change in HBP concentration is used as an early warning indicator for the prognosis risk of the patient with acute ST-segment elevation myocardial infarction.

5. The use according to claim 4, wherein the prognosis risk comprises one or a combined risk of reinfarction, acute heart failure, cardiogenic shock and stroke.

6. The use according to claim 3, wherein the kit is configured to detect the change in HBP concentration in the patient with acute ST-segment elevation myocardial infarction by detecting an in vitro sample from the patient.

7. The use according to claim 6, wherein the in vitro sample is plasma.
